# EUROPÄISCHE PATENTANMELDUNG

(11) **EP 4 318 014 A1**
(43) Veröffentlichungstag der Anmeldung: **07.02.2024**
(21) Anmeldenummer: 22189025.4
(22) Anmeldetag: 05.08.2022
(51) Int. Cl.: G01R 33/28, A61B 5/00

(54) **SYSTEM UND VERFAHREN ZUM EINSTELLEN EINER POSITION EINES PATIENTENTISCHS UND/ODER EINER POSITION EINES AUF DEM PATIENTENTISCH POSITIONIERTEN OBJEKTS IN EINEM MAGNETRESONANZTOMOGRAPHEN**

(71) Anmelder: Siemens Healthcare GmbH, 91052 Erlangen (DE)
(72) Erfinder: Beck, Thomas, 91077 Dormitz (DE); Helmecke, Sven, 90427 Nürnberg (DE)
(74) Vertreter: Siemens Healthineers Patent Attorneys

(57) **Zusammenfassung**

Die Erfindung geht aus von einem System umfassend eine Magnetresonanzvorrichtung und eine Überwachungseinheit,
wobei die Magnetresonanzvorrichtung umfasst:
- eine Scannereinheit, die zu einer Erfassung von medizinischen Magnetresonanzdaten ausgebildet ist,
- einen von der Scannereinheit zumindest teilweise umgebenen Patientenaufnahmebereich,
- eine Patientenlagerungsvorrichtung mit einem bewegbaren Patiententisch, wobei der Patiententisch in den Patientenaufnahmebereich einfahrbar ausgebildet ist,
- eine Positionserfassungseinheit, die zu einer Erfassung von Positionsdaten einer Position des Patiententischs und/oder eines auf dem Patiententisch positionierten Objekts ausgebildet ist, wobei die Positionierungseinheit eine Kamera umfasst, und
- eine Einstelleinheit, die zu einer Einstellung der Position des Patiententischs und/oder des auf dem Patiententisch positionierten Objekts anhand eines bereitgestellten Einstellparameters ausgebildet ist,

wobei die Überwachungseinheit zu einer Bereitstellung eines Einstellparameters zu einer Einstellung einer Position des Patiententischs und/oder des auf dem Patiententischs positionierten Objekts anhand der erfassten Positionsdaten ausgebildet ist.

## Beschreibung

Die vorliegende Erfindung betrifft ein System umfassend eine Magnetresonanzvorrichtung und eine externe Benutzerschnittstelle, wobei das System dazu ausgebildet ist, eine Positionierung eines Patiententischs und/oder eines auf dem Patiententisch positionierten Objekts einzustellen. Des Weiteren geht die Erfindung aus von einem Verfahren zu einem Einstellen einer Position eines Patiententischs und/oder eines auf dem Patiententischs positionierten Objekts. Ferner geht die Erfindung aus von einem Computerprogrammprodukt, welches ein Programm umfasst, um das Verfahren zu einem Einstellen einer Position eines Patiententischs und/oder eines auf dem Patiententischs positionierten Objekts auszuführen. Zudem geht die Erfindung aus von einem elektronisch lesbaren Datenträger mit dem Computerprogramm.

Für eine Magnetresonanzuntersuchung wird der Patient zunächst auf einer Patientenlagerungsvorrichtung, insbesondere einem Patiententisch der Patientenlagerungsvorrichtung, positioniert und anschließend der Patiententisch zusammen mit dem Patienten in einen Patientenaufnahmebereich der Magnetresonanzvorrichtung eingebracht und/oder eingefahren. Magnetresonanzvorrichtungen weisen hierzu bekannterweise einen Lasermarker auf, der über einer Einführöffnung des Patientenaufnahmebereichs angeordnet ist. Mittels des Lasermarkers kann der zu untersuchende Bereich des Patienten festgelegt und/oder markiert werden. Dabei wird der Patiententisch manuell so lange verschoben, bis der relevante Bereich und/oder der zu untersuchende Bereich im Fokus des Lasermarkers angeordnet ist. Anschließend wird der Patiententisch in den Patientenaufnahmebereich automatisch eingefahren, wobei die mittels des Lasermarkers markierte Position im Isozentrum der Magnetresonanzvorrichtung angeordnet ist. Jedoch ist es für ein ungeübtes und/oder unerfahrenes Bedienpersonal oft schwierig, den Patiententisch manuell in die korrekte Position für die Markierung zu positionieren.

Der vorliegenden Erfindung liegt insbesondere die Aufgabe zugrunde, eine Positionierung des Patiententischs innerhalb des Patientenaufnahmebereichs für einen Benutzer zu vereinfachen. Die Aufgabe wird durch die Merkmale der unabhängigen Ansprüche gelöst. Vorteilhafte Ausgestaltungen sind in den Unteransprüchen beschrieben.

Die Erfindung geht aus von einem System umfassend eine Magnetresonanzvorrichtung und eine Überwachungseinheit,
wobei die Magnetresonanzvorrichtung umfasst:
   - eine Scannereinheit, die zu einer Erfassung von medizinischen Magnetresonanzdaten ausgebildet ist,
   - einen von der Scannereinheit zumindest teilweise umgebenen Patientenaufnahmebereich,
   - eine Patientenlagerungsvorrichtung mit einem bewegbaren Patiententisch, wobei der Patiententisch in den Patientenaufnahmebereich einfahrbar ausgebildet ist,
   - eine Positionserfassungseinheit, die zu einer Erfassung von Positionsdaten einer Position des Patiententischs und/oder eines auf dem Patiententisch positionierten Objekts ausgebildet ist, wobei die Positionserfassungseinheit eine Kamera umfasst, und
   - eine Einstelleinheit, die zu einer Einstellung der Position des Patiententischs und/oder des auf dem Patiententischs positionierten Objekts anhand eines bereitgestellten Einstellparameters ausgebildet ist,
wobei die Überwachungseinheit zu einer Bereitstellung eines Einstellparameters zu einer Einstellung einer Position des Patiententischs und/oder des auf dem Patiententisch positionierten Objekts anhand der erfassten Positionsdaten ausgebildet ist.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Scannereinheit der Magnetresonanzvorrichtung umfasst bevorzugt eine Detektoreinheit, insbesondere eine Magneteinheit, zur Erfassung der medizinischen und/oder diagnostischen Bilddaten, insbesondere Magnetresonanzbilddaten. Hierbei umfasst die Scannereinheit, insbesondere die Magneteinheit, einen Grundmagneten, eine Gradientenspuleneinheit und eine Hochfrequenzantenneneinheit. Die Hochfrequenzantenneneinheit ist hierbei fest innerhalb der Scannereinheit angeordnet. Zudem kann die Magnetresonanzvorrichtung noch lokale Hochfrequenzspulen umfassen, die zur Erfassung von Magnetresonanzdaten um den zu untersuchenden Bereich eines Patienten angeordnet werden.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Das Gradienten-System ist zu einer Erzeugung von Magnetfeldgradienten, die für eine Ortskodierung während einer Bildgebung verwendet werden, ausgebildet.

Für eine Magnetresonanzuntersuchung wird der Patient, insbesondere der zu untersuchende Bereich des Patienten, innerhalb eines Patientenaufnahmebereichs der Magnetresonanzvorrichtung positioniert. Der Patientenaufnahmebereich ist dabei zumindest teilweise von der Scannereinheit umgeben, insbesondere zylinderförmig von der Scannereinheit umgeben. Innerhalb des Patientenaufnahmebereichs ist bevorzugt ein Field of View (FOV) und/oder ein Isozentrum der Magnetresonanzvorrichtung angeordnet. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereichs weist die Magnetresonanzvorrichtung eine Patientenlagerungsvorrichtung auf. Die Patientenlagerungsvorrichtung ist zu einer Lagerung und/oder Positionierung des Patienten für eine Magnetresonanzuntersuchung an dem Patienten ausgebildet. Die Patientenlagerungsvorrichtung weist bevorzugt einen bewegbaren Patiententisch auf, der insbesondere innerhalb des Patientenaufnahmebereichs der Magnetresonanzvorrichtung bewegbar ausgebildet ist. Für eine Magnetresonanzuntersuchung wird der Patient zunächst auf dem Patiententisch der Patientenlagerungsvorrichtung positioniert und anschließend der Patiententisch zusammen mit dem Patienten in den Patientenaufnahmebereich eingefahren, bis der zu untersuchende Bereich des Patienten innerhalb des Isozentrums positioniert ist.

Das auf dem Patiententisch positionierte Objekt kann beispielswiese einen Patienten, insbesondere den zu untersuchenden Bereich des Patienten, umfassen. Zudem kann das auf dem Patiententisch positionierte Objekt auch eine Zusatzeinheit, die für eine medizinische und/oder diagnostische Magnetresonanzuntersuchung um den Patienten, insbesondere um den zu untersuchenden Bereich des Patienten, angeordnet wird, wie beispielsweise eine lokale Hochfrequenzspule, umfassen. Des Weiteren kann das auf dem Patiententisch positionierte Objekt für beispielsweise Kalibrierungsmessungen ein Phantom usw. umfassen.

Die Positionserfassungseinheit ist bevorzugt dazu ausgebildet, eine Position des Patiententischs und/oder des auf dem Patiententisch positionierten Objekts zu erfassen. Bevorzugt kann dabei die Position des Patiententischs und/oder des auf dem Patiententisch positionierten Objekts bezüglich einer Referenzposition und/oder in Bezug zum Isozentrum der Scannereinheit erfasst werden. Die Positionserfassungseinheit umfasst bevorzugt eine Kamera. Vorzugsweise umfassen die Positionsdaten dabei Kameradaten und besonders vorteilhaft Videodaten. Bevorzugt weist hierbei die Positionserfassungseinheit, insbesondere die Kamera, einen Erfassungsbereich und/oder ein Sichtfeld auf, das auf einen Bereich direkt vor der Eingangsöffnung des Patientenaufnahmebereichs gerichtet ist.

Für eine Positionierung des Patiententischs und/oder des auf dem Patiententisch positionierten Objekts im Patientenaufnahmebereich, insbesondere im Isozentrum der Magnetresonanzvorrichtung, wird zunächst der Patiententisch und/oder das auf dem Patiententisch positionierte Objekt, insbesondere ein für die anstehende Untersuchung relevanter Bereich des Patiententischs und/oder des auf dem Patiententischs positionierten Objekts, in einer Referenzposition, die vorzugsweise einen zentralen Punkt im Sichtfeld der Kamera umfasst, positioniert. Bevorzugt ist die Positionserfassungseinheit, insbesondere die Kamera, derart ausgebildet, dass diese Referenzposition in den erfassten Positionsdaten markiert ist, beispielsweise durch ein Fadenkreuz. Für diese Positionierung des Patiententischs und/oder des auf dem Patiententisch positionierten Objekts in dieser Referenzposition werden mittels der Positionserfassungseinheit, insbesondere der Kamera, Positionsdaten erfasst. Diese Referenzposition weist einen definierten Abstand zum Isozentrum der Magnetresonanzvorrichtung auf, so dass nach einer Positionierung des Patiententischs und/oder des auf dem Patiententisch positionierten Objekts, insbesondere ein für die anstehende Untersuchung relevanter Bereich des Patiententischs und/oder des auf dem Patiententischs positionierten Objekts, in dieser Referenzposition der Patiententisch oder der Patiententisch zusammen mit dem auf dem Patiententisch positionierten Objekt schnell und einfach im Isozentrum positioniert werden kann. Für die Positionierung kann zudem auch der Patiententisch und/oder das auf dem Patiententisch positionierte Objekt, insbesondere ein für die anstehende Untersuchung relevanter Bereich des Patiententischs und/oder des auf dem Patiententischs positionierten Objekts, eine Markierung aufweisen.

Die Einstelleinheit der Magnetresonanzvorrichtung ist zu einer Einstellung einer Position des Patiententischs oder des Patiententisch zusammen mit dem auf dem Patiententisch positionierten Objekts ausgebildet. Die Einstelleinheit weist hierzu bevorzugt eine Antriebseinheit auf, die ein Antriebsmoment für eine Bewegung den Patiententisch generiert. Zudem kann die Einstelleinheit auch ein Rechenmodul und/oder einen Prozessor umfassen, um eine Bewegung des Patiententischs zu steuern. Weiterhin kann die Einstelleinheit auch eine entsprechende Software und/oder Computerprogramme zur Steuerung einer Bewegung und/oder Positionierung des Patiententischs umfassen. Der Einstellparameter umfasst bevorzugt einen Parameter zur Steuerung einer Bewegung des Patiententischs. Beispielsweise kann mittels der Einstellparameters eine Richtung und/oder eine Geschwindigkeit einer Bewegung des Patiententischs eingestellt und/oder gesteuert werden. Zudem kann der Einstellparameter auch einen Stoppparameter umfassen, der ein Stoppen der Bewegung des Patiententischs bewirkt, beispielsweise wenn der Patiententisch innerhalb der Referenzposition angeordnet ist oder auch in einer Gefahrensituation.

Die Überwachungseinheit ist dazu ausgebildet, einen Einstellparameter zur Einstellung einer Position des Patiententischs und/oder des auf dem Patiententisch positionierten Objekts anhand der erfassten Positionsdaten bereitzustellen. Dabei kann die Überwachungseinheit ein Rechenmodul und/oder einen Prozessor umfassen, um einen entsprechenden Einstellparameter anhand der erfassten Positionsdaten zu ermitteln, insbesondere automatisch und/oder selbsttätig zu ermitteln. Zudem kann die Überwachungseinheit auch eine entsprechende Software und/oder Computerprogramme zur Ermittlung eines Einstellparameter anhand der erfassten Positionsdaten umfassen, so dass die Überwachungseinheit zu einem automatischen Bereitstellen eines Einstellparameters zur Einstellung der Position des Patiententischs und/oder des auf dem Patiententischs positionierten Objekts anhand der mittels der Positionserfassungseinheit, insbesondere der Kamera, erfassten Positionsdaten ausgebildet ist.

Alternativ oder zusätzlich kann die Überwachungseinheit auch eine Benutzerschnittstelle umfassen, für ein manuelles Bereitstellen eines Einstellparameters zur Einstellung der Position des Patiententischs und/oder des auf dem Patiententischs positionierten Objekts. Dabei können durch die Benutzerschnittstelle, insbesondere eine Ausgabeeinheit der Benutzerschnittstelle, die erfassten Positionsdaten an einen Benutzer ausgegeben werden. Einstellparameter zur Einstellung einer Position des Patiententischs und/oder eines auf dem Patiententisch positionierten Objekts können dabei anhand der dargestellten Positionsdaten mittels einer Eingabeeinheit der Benutzerschnittstelle eingegeben werden.

Durch die Erfindung kann vorteilhaft eine einfache und schnelle Positionierung des Patiententischs und/oder des auf dem Patiententisch positionierten und/oder angeordneten Objekts in der Referenzposition bereitgestellt werden. Zudem kann hierbei eine Überwachung und/oder Kontrolle einer Positionierung des Patiententischs und/oder des auf dem Patiententisch positionierten und/oder angeordneten Objekts in der Referenzposition erfolgen. Mittels der Positionserfassungseinheit, insbesondere der Kamera, ist zudem eine kontinuierliche Überwachung, beispielsweise mittels eines Videostreams, der an die Überwachungseinheit übertragen und beispielsweise an einen Benutzer ausgegeben wird, möglich, die zudem eine einfache und schnelle Einstellung einer Position des Patiententischs und/oder des auf dem Patiententisch positionierten Objekts ermöglicht.

Zudem kann mittels der Positionserfassungseinheit, insbesondere der Kamera, weiterhin eine Positionierung des Patiententischs innerhalb des Patientenaufnahmebereichs überwacht werden und damit eine hoher Sicherheitsstandard bei der Positionierung des Patiententisch erreicht werden. Beispielsweise können derart während des Einfahrens des Patiententischs in den Patientenaufnahmebereich vom Patiententisch überhängende Teile, beispielsweise Kabel oder ein Arm des Patienten, erkannt werden und der Positionsvorgang gestoppt und/oder angehalten werden.

In einer vorteilhaften Weiterbildung des erfindungsgemäßen Systems kann es vorgesehen sein, dass die Magnetresonanzvorrichtung ein Schnittstellenmodul umfasst, das eine Schnittstelle aufweist und zu einem Aufbau einer Verbindung mi einem Schnittstellenmodul der Überwachungseinheit ausgebildet ist, und
die Überwachungseinheit eine externe Benutzerschnittstelle umfasst, mit:
- einem Schnittstellenmodul, das eine Schnittstelle aufweist und zu einem Aufbau einer Verbindung mit dem Schnittstellenmodul der Magnetresonanzvorrichtung ausgebildet ist,
- einer Ausgabeeinheit, die zu einer Ausgabe der Positionsdaten ausgebildet ist, und
- einer Eingabeeinheit, die zu einer Eingabe eines Einstellparameters zu einer Einstellung einer Positionierung des Patiententischs und/oder des auf dem Patiententisch positionierten Objekts ausgebildet ist.

Die Magnetresonanzvorrichtung umfasst bevorzugt eine interne Benutzerschnittstelle, die insbesondere eine Eingabeeinheit, beispielsweise eine Tastatur und/oder ein Touch-Display, und eine Ausgabeeinheit, wie beispielweise einen Monitor und/oder ein Display und/oder ein Touch-Display, umfasst. Die interne Schnittstelle ist in einem Betriebsmodus der Magnetresonanzvorrichtung permanent mit einer Steuereinheit und/oder weiteren Einheiten der Magnetresonanzvorrichtung verbunden, so dass ein Benutzer stets die interne Benutzerschnittstelle nutzen kann, ohne vorher eine Verbindung, insbesondere eine Datenverbindung, mit der Magnetresonanzvorrichtung, insbesondere der Steuereinheit der Magnetresonanzvorrichtung, herzustellen.

Das Schnittstellenmodul dagegen ist zu einem Aufbau einer Verbindung, insbesondere einer Datenverbindung mit einer externen Benutzerschnittstelle ausgebildet. Die externe Benutzerschnittstelle dient einem externen Nutzer, der sich nicht am Ort der Magnetresonanzvorrichtung aufhält, mit der Magnetresonanzvorrichtung Informationen und/oder Daten auszutauschen. Die externe Benutzerschnittstelle kann dabei außerhalb eines Gebäudes, in dem die Magnetresonanzvorrichtung angeordnet ist, angeordnet sein. Für einen Datenaustausch mit der Magnetresonanzvorrichtung muss der Nutzer hierzu zunächst eine Verbindung, insbesondere eine Datenverbindung, zwischen der externen Benutzerschnittstelle und der Magnetresonanzvorrichtung aufbauen und/oder herstellen. Die externe Benutzerschnittstelle weist hierzu ebenfalls ein Schnittstellenmodul mit einer Schnittstelle auf. Für den Aufbau einer Datenverbindung werden bevorzugt Verbindungsaufbaudaten, beispielswiese Zugangsdaten usw., zwischen dem Schnittstellenmodul der Magnetresonanzvorrichtung und dem Schnittstellenmodul der externen Benutzerschnittstelle ausgetauscht. Die externe Benutzerschnittstelle weist bevorzugt eine Ausgabeeinheit, die insbesondere einen Monitor oder ein Display umfasst, zur Ausgabe der mittels der Positionserfassungseinheit erfassten Positionsdaten, und eine Eingabeeinheit, beispielsweise ein Touch-Display, eine Tastatur und/oder eine Computermaus usw., auf. Zudem kann die externe Benutzerschnittstelle weitere Einheiten, wie beispielsweise einen Prozessor und/oder eine Speichereinheit usw. umfassen.

Durch diese Ausgestaltung der Erfindung kann vorteilhaft ein Positionierungsvorgang des Patiententischs von einem Experten und/oder einem in Positioniervorgängen erfahrenen Benutzer von der externen Benutzerschnittstelle aus überwacht und/oder gesteuert werden. Insbesondere kann durch eine derartige Vorgehensweise Zeit gegenüber einer von einem unerfahrenen Benutzer vor Ort durchgeführten Positionierung des Patiententischs eingespart werden. Ein weiterer Vorteil ist, dass auch ein unerfahrenes und/oder ungeübtes Personal bei der Vorbereitung und/oder Durchführung einer Magnetresonanzvorrichtung vorteilhaft unterstützt wird.

In einer vorteilhaften Weiterbildung des erfindungsgemäßen Systems kann es vorgesehen sein, dass die Scannereinheit eine Frontseite und eine Heckseite mit jeweils einer Einführöffnung für den Patientenaufnahmebereich umfasst und die Positionserfassungseinheit an der Frontseite und/oder an der Heckseite der Scannereinheit oberhalb der Einführöffnung angeordnet ist. Vorzugsweise ist dabei die Positionserfassungseinheit, insbesondere die Kamera, derart oberhalb der Einführöffnung angeordnet, dass das Sichtfeld der Positionserfassungseinheit, insbesondere der Kamera, senkrecht nach unten ausgerichtet ist. Dieser Anordnung der Positionserfassungseinheit weist den Vorteil auf, dass die Positionserfassungseinheit, insbesondere die Kamera, eine ungehinderte Sicht auf den Patiententisch und damit auch auf das auf dem Patiententisch positionierte Objekt in der Referenzposition hat.

In einer vorteilhaften Weiterbildung des erfindungsgemäßen Systems kann es vorgesehen sein, dass die Positionserfassungseinheit, insbesondere die Kamera, eine Datenschnittstelle aufweist für eine Verbindung mit der Einstelleinheit. Die Datenschnittstelle umfasst bevorzugt eine universelle Datenschnittstelle, wie beispielsweise eine USB-Schnittstelle und/oder eine HDMI-Schnittstelle usw. Derart kann die Positionserfassungseinheit auch besonders einfach nachträglich in bereits bestehende Magnetresonanzvorrichtungen zur Erfassung von Positionsdaten des Patiententischs und/oder des auf dem Patiententisch positionierten Objekts integriert werden. Zudem kann derart auch für die Erfassung der Positionsdaten eine herkömmliche Kamera verwendet werden und damit eine kostengünstige Positionserfassungseinheit bereitgestellt werden.

In einer vorteilhaften Weiterbildung des erfindungsgemäßen Systems kann es vorgesehen sein, dass die Positionserfassungseinheit ein Markierungselement aufweist, das zu einer Markierung eines relevanten Bereichs des Patiententischs und/oder des auf dem Patiententisch positionierten Objekts ausgebildet ist. Das Markierungselement kann dabei ein in die Optik der Positionserfassungseinheit, insbesondere der Kamera, integriertes Markierungselement, wie beispielsweise ein Fadenkreuz, umfassen. Zudem kann das Markierungselement auch ein den erfassten Positionsdaten überlagertes Markierungselement umfassen. Das Markierungselement kann zudem auch ein Laserelement umfassen, dass eine Markierung, insbesondere eine Lasermarkierung direkt auf den Patiententisch und/oder auf das auf dem Patiententisch positionierte Objekt während einer Erfassung der Positionsdaten mittels der Positionserfassungseinheit, insbesondere der Kamera, projiziert. Derart kann in besonders einfacher Art und Weise ein Referenzpunkt für die anschließende Positionierung des Patiententischs und/oder des auf dem Patiententisch positionierten Objekts innerhalb des Patientenaufnahmebereichs, insbesondere innerhalb des Isozentrums der Magnetresonanzvorrichtung, erfasst werden.

In einer vorteilhaften Weiterbildung des erfindungsgemäßen Systems kann es vorgesehen sein, dass die Positionserfassungseinheit, insbesondere die Kamera, eine Kalibriervorrichtung aufweist. Eine Kalibrierung der Positionserfassungseinheit, insbesondere der Kamera, wird insbesondere bei einer Installation der Magnetresonanzvorrichtung und/oder einer Installation der Positionserfassungseinheit durchgeführt. Zudem kann eine Kalibrierung der Positionserfassungseinheit, insbesondere der Kamera, auch in regelmäßigen zeitlichen Abständen durchgeführt werden, um eine Ausrichtung der Positionserfassungseinheit, insbesondere der Kamera, zu überprüfen. Die Kalibriervorrichtung umfasst beispielsweise einen Spiegel, der in einer definierten Position auf dem Patiententisch positioniert ist. Zudem kann die Kalibriervorrichtung auch eine Lasermarkierung umfassen. Die Kalibrierung der Kamera kann dabei von einem Benutzer, insbesondere einem externen Benutzer, mittels der Benutzerschnittstelle, insbesondere mittels der externen Benutzerschnittstelle, durchgeführt werden. Derart kann eine einfache und schnelle Kalibrierung von einem erfahrenen Benutzer durchgeführt werden und damit auch eine Genauigkeit der Positionserfassungseinheit gewährleistet werden.

Des Weiteren geht die Erfindung aus von einem Verfahren zu einem Einstellen einer Position eines Patiententischs und/oder eines auf dem Patiententischs positionierten Objekts in einem System, umfassend die folgenden Verfahrensschritte:
- S1: Erfassen von Positionsdaten des Patiententischs und/oder des auf dem Patiententisch positionierten Objekts mittels einer Positionserfassungseinheit einer Magnetresonanzvorrichtung, wobei die Positionsdaten zumindest teilweise Videodaten umfassen,
- S2: Bereitstellen der erfassten Positionsdaten an eine Überwachungseinheit,
- S3: Bestimmen zumindest eines Einstellparameters für eine Einstellung einer Position des Patiententischs und/oder des auf dem Patiententisch positionierten Objekts mittels der Überwachungseinheit anhand der erfassten Positionsdaten,
- S4: Bereitstellen des zumindest einen Einstellparameters an eine Einstelleinheit, und
- S5: Einstellen der Position des Patiententischs und/oder des auf dem Patiententisch positionierten Objekts mittels der Einstelleinheit anhand des zumindest einen bereitgestellten Einstellparameters.

Das Bereitstellen der erfassten Positionsdaten kann ein Übertragen der erfassten Positionsdaten von der Positionserfassungseinheit an die Überwachungseinheit umfassen. Ebenso umfasst auch das Bereitstellen des zumindest einen Einstellparameters ein Übertragen des zumindest einen Einstellparameters von der Überwachungseinheit an die Einstelleinheit. Umfasst die Überwachungseinheit eine interne Einheit, insbesondere eine von der Magnetresonanzvorrichtung umfasste Einheit, kann das Bereitstellen der erfassten Positionsdaten eine Datenübertragung von der Positionserfassungseinheit an die Überwachungseinheit mittels einer internen, bereits vorhandenen Datenverbindung der Magnetresonanzvorrichtung erfolgen. Zudem kann das Bereitstellen des zumindest einen Einstellparameters eine Datenübertragung von der Überwachungseinheit an die Einstelleinheit mittels einer internen, bereits vorhandenen Datenverbindung der Magnetresonanzvorrichtung erfolgen.

Umfasst die Überwachungseinheit dagegen eine externe Einheit, insbesondere eine externe Benutzerschnittstelle, ist für das Bereitstellen, insbesondere das Übertragen, der Positionsdaten eine Datenverbindung zwischen der Magnetresonanzvorrichtung, beispielsweise eine Steuereinheit der Magnetresonanzvorrichtung, und der externen Benutzerschnittstelle erforderlich. Auch für das Bereitstellen des zumindest einen Einstellparameters ist eine Datenverbindung zwischen der Magnetresonanzvorrichtung, beispielsweise eine Steuereinheit der Magnetresonanzvorrichtung, und der externen Benutzerschnittstelle erforderlich. Hierzu weisen bevorzugt die externe Benutzerschnittstelle und auch die Magnetresonanzvorrichtung ein entsprechendes Schnittstellenmodul auf, um die Datenverbindung zwischen der Magnetresonanzvorrichtung und der externen Benutzerschnittstelle aufzubauen.

Durch das erfindungsgemäße Verfahren kann vorteilhaft ein Positionierungsvorgang des Patiententischs von einem Experten und/oder einem in Positioniervorgängen erfahrenen Benutzer von der externen Benutzerschnittstelle aus überwacht und/oder gesteuert werden. Insbesondere kann durch eine derartige Vorgehensweise Zeit gegenüber einer von einem unerfahrenen Benutzer vor Ort durchgeführten Positionierung des Patiententischs eingespart werden. Ein weiterer Vorteil ist, dass auch ein unerfahrenes und/oder ungeübtes Personal bei der Vorbereitung und/oder Durchführung einer Magnetresonanzvorrichtung vorteilhaft unterstützt wird.

Zudem kann vorteilhaft eine einfache und schnelle Positionierung des Patiententischs und/oder des auf dem Patiententisch positionierten und/oder angeordneten Objekts in der Referenzposition bereitgestellt werden. Zudem kann hierbei eine Überwachung und/oder Kontrolle einer Positionierung des Patiententischs und/oder des auf dem Patiententisch positionierten und/oder angeordneten Objekts in der Referenzposition erfolgen. Mittels der Positionserfassungseinheit, insbesondere der Kamera, ist zudem eine kontinuierliche Überwachung, beispielsweise mittels eines Videostreams, der an die Überwachungseinheit übertragen und beispielsweise an einen Benutzer ausgegeben wird, möglich, die zudem eine einfache und schnelle Einstellung einer Position des Patiententischs und/oder des auf dem Patiententisch positionierten Objekts ermöglicht.

Zudem kann mittels der Positionserfassungseinheit, insbesondere der Kamera, weiterhin eine Positionierung des Patiententischs innerhalb des Patientenaufnahmebereichs überwacht werden und damit eine hoher Sicherheitsstandard bei der Positionierung des Patiententisch erreicht werden. Beispielsweise können derart während des Einfahrens des Patiententischs in den Patientenaufnahmebereich vom Patiententisch überhängende Teile, beispielsweise Kabel oder ein Arm des Patienten, erkannt werden und der Positionsvorgang gestoppt und/oder angehalten werden.

Die Vorteile des erfindungsgemäßen Verfahrens zu einem Einstellen einer Position eines Patiententischs und/oder eines auf dem Patiententisch positionierten Objekts entsprechen im Wesentlichen den Vorteilen des erfindungsgemäßen Systems, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Verfahrens kann es vorgesehen sein, dass die Verfahrensschritte S1 bis S5 so oft wiederholt werden, bis der Patiententisch und/oder das auf dem Patiententischs positionierte Objekt in einer Referenzposition angeordnet ist. Die Referenzposition weist einen definierten und/oder vorbestimmten Abstand zum Isozentrum der Magnetresonanzvorrichtung auf. Für die Positionierung des Patiententischs und/oder des zu untersuchenden Objekts wird insbesondere ein für die anstehende Untersuchung relevanter Bereich des Patiententischs und/oder des auf dem Patiententischs positionierten Objekts in dieser Referenzposition positioniert. Dieser relevante Bereich des Patiententischs und/oder des auf dem Patiententisch positionierten Objekts kann zudem auch durch eine Markierung, die in dem relevanten Bereich am Patiententisch und/oder am auf dem Patiententisch angeordneten Objekt angeordnet ist, gekennzeichnet sein.

Diese Ausgestaltung der Erfindung weist den Vorteil auf, dass ein Positionierungsvorgang zur Positionierung des Patiententischs und/oder des auf dem Patiententisch positionierten Objekts direkt überwacht, insbesondere von extern überwacht, werden kann. Zudem kann bei unerwünschten Bewegungen des Patiententischs gezielt gegengesteuert werden, beispielsweise durch eine Auswahl entsprechender Einstellparameter.

In einer vorteilhaften Weiterbildung des erfindungsgemäßen Verfahrens kann es vorgesehen sein, dass während eines gesamten Einfahrvorgangs des Patiententischs in den Patientenaufnahmebereich von der Positionserfassungseinheit Positionsdaten erfasst und der Überwachungseinheit bereitgestellt werden. Damit kann der Einfahrvorgang des Patiententischs, bis dieser in seiner Zielposition innerhalb des Isozentrums der Magnetresonanzvorrichtung angeordnet ist, vorteilhaft überwacht werden. Insbesondere kann derart automatisch mittels der Überwachungseinheit und/oder manuell von einem Benutzer mittels der Überwachungseinheit der Einfahrvorgang überwacht werden und dabei mögliche Gefahren, wie beispielsweise herabhängende Kabel und/oder ein Arm oder ein Bein des Patienten ragt über die Patientenlagerungsvorrichtung hinaus usw. erkannt werden. Dies ermöglicht es, in einer Gefahrensituation die Bewegung des Patiententischs zu stoppen und/oder weitere Maßnahmen zur Behebung der Gefahr einzuleiten und damit eine Sicherheit für den Patienten während einer Magnetresonanzuntersuchung vorteilhaft zu erhöhen.

In einer vorteilhaften Weiterbildung des erfindungsgemäßen Verfahrens kann es vorgesehen sein, dass die Positionserfassungseinheit während einer Magnetresonanzmessung deaktiviert wird. Dies weist den Vorteil auf, dass eine unterwünschte Interaktion zwischen der Positionserfassungseinheit, insbesondere der Kamera, und der Magnetresonanzvorrichtung, insbesondere der Hochfrequenzantenneneinheit, vorteilhaft verhindert werden kann. Damit einhergehend kann die Positionserfassungseinheit, insbesondere die Kamera, ohne besondere Schirmung und damit Bauteile und Kosten sparend an der Magnetresonanzvorrichtung, insbesondere der Scannereinheit der Magnetresonanzvorrichtung, angeordnet werden.

In einer vorteilhaften Weiterbildung des erfindungsgemäßen Verfahrens kann es vorgesehen sein, dass die Überwachungseinheit ein Rechenmodul aufweist, das anhand der bereitgestellten Positionsdaten automatisch und/oder selbsttätig den zumindest einen Einstellparameter ermittelt. Die Überwachungseinheit kann dabei Rechenmodul und/oder einen Prozessor umfassen. So kann insbesondere die Überwachungseinheit dazu ausgebildet sein, computerlesbare Instruktionen auszuführen. Insbesondere kann die Überwachungseinheit eine Speichereinheit umfassen, wobei auf der Speichereinheit computerlesbare Informationen gespeichert sind, wobei die Überwachungseinheit dazu ausgebildet ist, die computerlesbaren Informationen von der Speichereinheit zu laden und die computerlesbaren Informationen auszuführen. Die Komponenten der Überwachungseinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Derart kann vorteilhaft eine schnelle und automatische Positionierung des Patiententischs und/oder des auf dem Patiententisch positionierten Objekts bereitgestellt werden. Insbesondere bei Messungen mit Phantomen kann dies von Vorteil sein.

In einer vorteilhaften Weiterbildung des erfindungsgemäßen Verfahrens kann es vorgesehen sein, dass die Überwachungseinheit eine externe Benutzerschnittstelle mit einem Schnittstellenmodul umfasst, wobei das Schnittstellenmodul zu einem Übertragen der Positionsdaten und/oder des zumindest einen Einstellparameters eine Datenverbindung mit der Magnetresonanzvorrichtung eingeht.

Durch diese Ausgestaltung der Erfindung kann vorteilhaft ein Positionierungsvorgang des Patiententischs von einem Experten und/oder einem in Positioniervorgängen erfahrenen Benutzer, insbesondere einen externen Benutzer, von der externen Benutzerschnittstelle aus überwacht und/oder gesteuert werden. Insbesondere kann durch eine derartige Vorgehensweise Zeit gegenüber einer von einem unerfahrenen Benutzer vor Ort durchgeführten Positionierung des Patiententischs eingespart werden und damit auch ein unerfahrenes Personal unterstützt werden.

Des Weiteren geht die Erfindung aus von einem Computerprogrammprodukt, welches ein Programm umfasst und direkt in einem Speicher einer programmierbaren Steuereinheit ladbar ist, mit Programmmitteln, um ein Verfahren zu einem Einstellen einer Position eines Patiententischs und/oder eines auf dem Patiententisch positionierten Objekts zu steuern, wenn das Programm in der Steuereinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Das erfindungsgemäße Computerprogrammprodukt ist direkt in einen Speicher einer programmierbaren Recheneinheit und/oder Steuereinheit ladbar und weist Programmcode-Mittel auf, um ein erfindungsgemäßes Verfahren auszuführen, wenn das Computerprogrammprodukt in der Recheneinheit und/oder Steuereinheit ausgeführt wird. Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist derart konfiguriert, dass es mittels der Recheneinheit und/oder Steuereinheit die erfindungsgemäßen Verfahrensschritte ausführen kann. Die Recheneinheit und/oder Steuereinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor einer lokalen Recheneinheit und/oder Steuereinheit geladen werden kann, der mit der Magnetresonanzvorrichtung direkt verbunden oder als Teil ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit und/oder Steuereinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuerung und/oder Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäßes System mit einer Magnetresonanzvorrichtung und einer Positionserfassungseinheit in einer schematischen Darstellung,
- Fig. 2: mittels der Positionserfassungseinheit erfasste Positionsdaten,
- Fig. 3: eine Kalibriervorrichtung der Positionserfassungseinheit, und
- Fig. 4: ein erfindungsgemäßes Verfahren zu einem Einstellen einer Position eines Patiententischs und/oder eines auf dem Patiententisch positionierten Objekts.

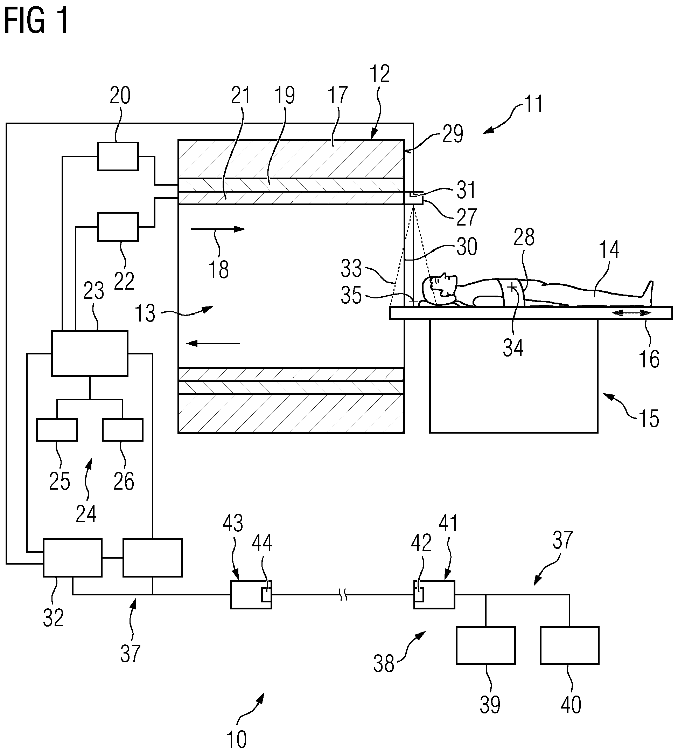

In Fig. 1 ist ein System 10 mit einer Magnetresonanzvorrichtung 11 schematisch dargestellt. Die Magnetresonanzvorrichtung 11 umfasst eine von einer Magneteinheit gebildeten Scannereinheit 12. Zudem weist die Magnetresonanzvorrichtung 11 einen Patientenaufnahmebereich 13 auf zu einer Aufnahme eines Patienten 14. Der Patientenaufnahmebereich 13 im vorliegenden Ausführungsbeispiel ist zylinderförmig ausgebildet und in einer Umfangsrichtung von der Scannereinheit 12, insbesondere von der Magneteinheit, zylinderförmig umgeben. Grundsätzlich ist jedoch eine davon abweichende Ausbildung des Patientenaufnahmebereichs 13 jederzeit denkbar. Der Patient 14 kann mittels einer Patientenlagerungsvorrichtung 15 der Magnetresonanzvorrichtung 11 in den Patientenaufnahmebereich 13 geschoben und/oder gefahren werden. Die Patientenlagerungsvorrichtung 15 weist hierzu einen innerhalb des Patientenaufnahmebereichs 13 bewegbar ausgestalteten Patiententisch 16 auf. Insbesondere ist hierbei der Patiententisch 16 in Richtung einer Längserstreckung des Patientenaufnahmebereichs 13 und/oder in z-Richtung bewegbar gelagert.

Die Scannereinheit 12, insbesondere die Magneteinheit, umfasst einen supraleitenden Grundmagneten 17 zu einem Erzeugen eines starken und insbesondere konstanten Grundmagnetfelds 18. Weiterhin weist die Scannereinheit 12, insbesondere die Magneteinheit, eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 20 der Magnetresonanzvorrichtung 11 gesteuert. Die Scannereinheit 12, insbesondere die Magneteinheit, umfasst weiterhin eine Hochfrequenzantenneneinheit 21 zu einer Anregung einer Polarisation, die sich in dem von dem Grundmagneten 17 erzeugten Grundmagnetfeld 18 einstellt. Die Hochfrequenzantenneneinheit 21 wird von einer Hochfrequenzantennensteuereinheit 22 der Magnetresonanzvorrichtung 11 gesteuert und strahlt hochfrequente Magnetresonanzsequenzen in den Patientenaufnahmebereich 13 der Magnetresonanzvorrichtung 11 ein.

Zu einer Steuerung des Grundmagneten 17, der Gradientensteuereinheit 20 und zur Steuerung der Hochfrequenzantennensteuereinheit 22 weist die Magnetresonanzvorrichtung 11 eine Systemsteuereinheit 23 auf. Die Systemsteuereinheit 23 steuert zentral die Magnetresonanzvorrichtung 11, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 11 eine interne Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Ausgabeeinheit 25, beispielsweise auf zumindest einem Monitor, der internen Benutzerschnittstelle 24 für ein vor Ort an der Magnetresonanzvorrichtung tätiges medizinisches Bedienpersonal angezeigt werden. Weiterhin weist die interne Benutzerschnittstelle 24 eine Eingabeeinheit 26 auf, mittels der Informationen und/oder Parameter während eines Messvorgangs von dem medizinischen Bedienpersonal eingegeben werden können. Die interne Benutzerschnittstelle 24 der Magnetresonanzvorrichtung 11 ist permanent mit der Systemsteuereinheit 23 und/oder weiteren Einheiten der Magnetresonanzvorrichtung 11 verbunden, so dass der Benutzer stets die interne Benutzerschnittstelle 24 nutzen kann, ohne vorher eine Verbindung, insbesondere eine Datenverbindung, mit der Magnetresonanzvorrichtung 11, insbesondere der Systemsteuereinheit 23 der Magnetresonanzvorrichtung 11, herzustellen.

Die Magnetresonanzvorrichtung 11 weist weiterhin eine Positionserfassungseinheit 27 auf, die zu einer Erfassung von Positionsdaten einer Position des Patiententischs 16 und/oder eines auf dem Patiententisch 16 positionierten Objekts 28 ausgebildet ist (Fig. 1). Das auf dem Patiententisch 27 positionierte Objekt 28 kann ein Patient 14, insbesondere ein zu untersuchender Bereich eines Patienten 14, und/oder eine lokale Hochfrequenzspule und/oder ein Phantom usw. sein. Die Positionserfassungseinheit 27 umfasst eine Kamera. Die Positionserfassungseinheit 27, insbesondere die Kamera, ist an einer Frontseite 29 der Scannereinheit 12 angeordnet. Insbesondere ist die Kamera oberhalb einer Einführöffnung 30 des Patientenaufnahmebereichs 13 an der Frontseite 29 der Scannereinheit 12 angeordnet. Die Positionserfassungseinheit 27, insbesondere die Kamera, weist dabei eine Datenschnittstelle 31 auf für eine Verbindung, insbesondere eine Datenverbindung, mit der Systemsteuereinheit 23 und/oder einer Einstelleinheit 32 der Magnetresonanzvorrichtung 11. Die Datenschnittstelle 31 umfasst bevorzugt eine universelle Datenschnittstelle 31, wie beispielsweise eine USB-Schnittstelle und/oder eine HDMI-Schnittstelle usw. Alternativ hierzu kann die Positionserfassungseinheit 27, insbesondere die Kamera, auch an einer Heckseite der Scannereinheit 12, insbesondere oberhalb einer Einführöffnung des Patientenaufnahmebereichs 13 an der Heckseite der Scannereinheit 12, angeordnet sein.

Die Positionserfassungseinheit 27, insbesondere die Kamera, weist bevorzugt einen Erfassungsbereich und/oder ein Sichtfeld 33 auf, das auf einen Bereich direkt vor der Einführöffnung 30 gerichtet ist (Fig. 1). Der Patiententisch 16 und/oder das auf dem Patiententisch 16 positionierte Objekt 28 wird für eine Positionierung innerhalb des Isozentrums der Magnetresonanzvorrichtung 11 zunächst in einer Referenzposition positioniert, die bevorzugt im Sichtfeld 33 der Kamera angeordnet ist. Insbesondere wird hierbei ein relevanter Punkt und/oder ein relevanter Bereich des Patiententischs 16 und/oder des auf dem Patiententisch 16 positionierte Objekts 28 in dieser Referenzposition positioniert und/oder angeordnet. Dieser relevante Punkt und/oder ein relevanter Bereich des Patiententischs 16 und/oder des auf dem Patiententisch 16 positionierte Objekts 28 ist im vorliegenden Ausführungsbeispiel mittels eines Markierungselements 34 gekennzeichnet. Die Referenzposition weist einen definierten Abstand zum Isozentrum der Magnetresonanzvorrichtung 11 auf. Um den Patiententisch 16 und/oder das auf dem Patiententisch 16 positionierte Objekt 28 in der Referenzposition zu positionieren, werden von der Positionserfassungseinheit 27 Positionsdaten erfasst.

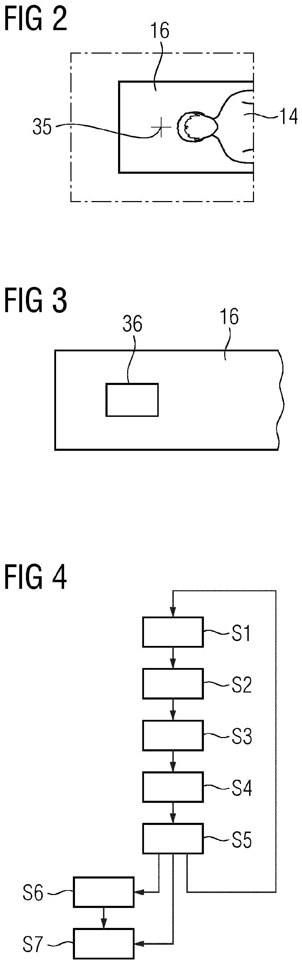

Für eine genaue Erfassung von Positionsdaten weist die Positionserfassungseinheit 27 ein Markierungselement 35 auf, dass zu einer Markierung des relevanten Bereichs des Patiententischs 16 und/oder des auf dem Patiententisch 16 positionierten Objekts 28 ausgebildet ist, wie dies in Fig. 2 zu sehen ist. In Fig. 2 sind mittels der Positionserfassungseinheit 27, insbesondere der Kamera, erfasste Positionsdaten dargestellt. In den Positionsdaten ist das Markierungselement 35 der Positionserfassungseinheit 27 zu sehen. Zu dem ist der Patiententisch 16 mit dem auf dem Patiententisch 16 positionierten Patienten 14 zu erkennen. Das Markierungselement 35 der Positionserfassungseinheit 27 kann dabei ein in die Optik der Positionserfassungseinheit 27, insbesondere der Kamera, integriertes Markierungselement 35, wie beispielsweise ein Fadenkreuz, umfassen. Zudem kann das Markierungselement 35 der Positionserfassungseinheit 27 auch ein den Positionsdaten überlagertes Markierungselement 35 umfassen. Das Markierungselement 35 der Positionserfassungseinheit 27 kann zudem auch ein Laserelement umfassen, dass eine Markierung, insbesondere eine Lasermarkierung direkt auf den Patiententisch 16 und/oder auf das auf dem Patiententisch 16 positionierte Objekt 28 projiziert.

Die Positionserfassungseinheit 27, insbesondere die Kamera, weist zudem eine Kalibriervorrichtung 36 auf (Fig. 3). Die Kalibriervorrichtung 36 umfasst im vorliegenden Ausführungsbeispiel beispielsweise einen Spiegel, der für eine Kalibrierung der Positionserfassungseinheit 27, insbesondere der Kamera, in einer definierten Position auf dem Patiententisch 16 positioniert ist. Zudem kann die Kalibriervorrichtung 36 auch die Lasermarkierung umfassen. Hierbei wird die Positionserfassungseinheit 27, insbesondere die Kamera, anhand eines reflektiert Optik, insbesondere eine Linse, der Kamera kalibriert. Dabei sollte die reflektierte Optik, insbesondere ein Zentrum der Linse, mit dem Markierungselement 35 der Kamera übereinstimmen.

Die Magnetresonanzvorrichtung 11 weist zudem eine Einstelleinheit 32 auf, die zu einer Einstellung der Position des Patiententischs 16 und/oder des auf dem Patiententischs 16 positionierten Objekts 28 anhand eines bereitgestellten Einstellparameters ausgebildet ist (Fig. 1). Die Einstelleinheit 32 weist hierzu bevorzugt eine nicht näher dargestellte Antriebseinheit auf, die ein Antriebsmoment für eine Bewegung des Patiententischs 16 generiert. Zudem kann die Einstelleinheit 32 auch ein Rechenmodul und/oder einen Prozessor umfassen, um eine Bewegung des Patiententischs 16 und damit auch eine Positionierung des Patiententischs 16 zu steuern. Weiterhin kann die Einstelleinheit 32 auch eine entsprechende Software und/oder Computerprogramme zur Steuerung einer Bewegung und/oder Positionierung des Patiententischs 16 umfassen.

Das System 10 umfasst des Weiteren eine Überwachungseinheit 37, die zu einer Bereitstellung eines Einstellparameters zur Einstellung der Position des Patiententischs 16 und/oder des auf dem Patiententisch 16 positionierten Objekts 28 anhand der erfassten Positionsdaten ausgebildet ist. Die Überwachungseinheit 37 umfasst bevorzugt ein Rechenmodul und/oder einen Prozessor, um einen entsprechenden Einstellparameter anhand der erfassten Positionsdaten zu ermitteln. Das Rechenmodul und/oder der Prozessor ist bevorzugt von der Magnetresonanzvorrichtung 11 umfasst. Zudem kann die Überwachungseinheit 37 auch eine entsprechende Software und/oder Computerprogramme zur Ermittlung eines Einstellparameter anhand der erfassten Positionsdaten umfassen, so dass die Überwachungseinheit 37 zu einem automatischen Bereitstellen eines Einstellparameters zur Einstellung der Position des Patiententischs 16 und/oder des auf dem Patiententisch 16 positionierten Objekts 38 ausgebildet ist.

Die Überwachungseinheit 37 umfasst des Weiteren eine externe Benutzerschnittstelle 38 (Fig. 1), wobei die externe Benutzerschnittstelle 38 eine Ausgabeeinheit 39 umfasst. Mittels der Ausgabeeinheit 38 kann eine Ausgabe der mittels der Positionserfassungseinheit 27 erfassten Positionsdaten für einen externen Benutzer erfolgen. Zudem umfasst die externe Benutzerschnittstelle 38 eine Eingabeeinheit 40. Mittels der Eingabeeinheit 40 können Einstellparameter zu Einstellung einer Position des Patiententischs 16 und/oder des auf dem Patiententisch 16 positionierten Objekts 28 von einem externen Benutzer eingegeben werden. Die externe Benutzerschnittstelle 38 umfasst des Weiteren ein Schnittstellenmodul 41 mit einer Schnittstelle 42, das zu einem Aufbau einer Verbindung, insbesondere einer Datenverbindung, mit der Magnetresonanzvorrichtung 11 ausgebildet ist. Ebenso weist hierzu auch die Magnetresonanzvorrichtung 11 ein Schnittstellenmodul 43 mit einer Schnittstelle 44 auf, wobei die Schnittstelle 44 zu einem Aufbau einer Verbindung, insbesondere einer Datenverbindung mit dem Schnittstellenmodul 41 der externen Benutzerschnittstelle 38 ausgebildet ist.

Die dargestellte Magnetresonanzvorrichtung 11 kann selbstverständlich weitere Komponenten umfassen, die Magnetresonanzvorrichtungen 11 gewöhnlich aufweisen. Eine allgemeine Funktionsweise einer Magnetresonanzvorrichtung 11 ist zudem dem Fachmann bekannt, so dass auf eine detaillierte Beschreibung der weiteren Komponenten verzichtet wird.

In Fig. 4 ist ein erfindungsgemäßes Verfahren zu einem Einstellen einer Position des Patiententischs 16 und/oder des auf dem Patiententisch 16 positionierten Objekts 28 dargestellt. Das Verfahren wird mittels des Systems 10 mit der Magnetresonanzvorrichtung 11 und der Überwachungseinheit 37 ausgeführt. Zudem wird das Verfahren von einer Steuereinheit 45 der Magnetresonanzvorrichtung 11 gesteuert, wobei die Steuereinheit 45 hierzu ein Rechenmodul und/oder einen Prozessor aufweist. Zudem umfasst die Steuereinheit 45 entsprechende Steuerprogramme und/oder Steuersoftware, die bei einer Ausführung zu einer Steuerung einer Einstellung einer Position eines Patiententischs 16 und/oder eines auf dem Patiententisch 16 positionierten Objekts 28 ausgebildet ist. Die Steuereinheit 45 kann dabei von der Einstelleinheit 32 und/oder der Systemsteuereinheit 23 umfasst sein. Zudem ist es auch denkbar, dass die Steuereinheit 45 separat zur Einstelleinheit 32 und/oder der Systemsteuereinheit 23 ausgebildet ist.

Zu Beginn des Verfahrens befindet sich bereits das Objekt 28 auf dem Patiententisch 16 positioniert. Das Objekt 28 kann beispielsweise den Patienten 14 umfassen, der für eine Magnetresonanzuntersuchung auf dem Patiententisch 16 positioniert ist. Zudem kann das Objekt 28 auch eine lokale Hochfrequenzspule umfassen, die um den zu untersuchenden Bereich des Patienten 14 für eine Erfassung von Magnetresonanzdaten angeordnet ist. Des Weiteren kann das Objekt 28 ein Phantom für beispielsweise eine Justagemessung usw. umfassen, das in einer definierten Position auf dem Patiententisch 16 angeordnet ist. Ein für die anstehende Messung relevanter Bereich des Patiententischs 16 und/oder des auf dem Patiententisch 16 positionierten Objekts 28 kann zudem auch mit dem Markierelement 34 gekennzeichnet sein.

In einem ersten Verfahrensschritt S1 erfolgt hierbei ein Erfassen von Positionsdaten des Patiententischs 16 und/oder des auf dem Patiententisch 16 positionierten Objekts 28 mittels der Positionserfassungseinheit 27, insbesondere der Kamera. Die erfassten Positionsdaten umfassen hierbei Videodaten. Bevorzugt werden hierbei während des gesamten Positionierungsvorgangs des Patiententischs 16 und/oder des auf dem Patiententisch 16 positionierten Objekts 28 Positionsdaten, insbesondere Videodaten, erfasst. Insbesondere werden hierbei Positionsdaten von der Referenzposition erfasst und mittels der Positionsdaten eine Position eines relevanten und/oder mittels des Markierungselements 34 markierten Bereichs des Patiententischs 16 und/oder des auf dem Patiententischs 16 positionierten Objekts 28 im Umfeld und/oder innerhalb der Referenzposition bestimmt.

In einem an den ersten Verfahrensschritt S1 anschließenden zweiten Verfahrensschritt S2 erfolgt ein Bereitstellen der erfassten Positionsdaten an die Überwachungseinheit 37. Das Bereitstellen der Positionsdaten kann dabei ein Bereitstellen der Positionsdaten an ein Rechenmodul der Überwachungseinheit 37 umfassen. Dabei kann das Bereitstellen eine Datenübertragung von der Positionserfassungseinheit 27 an die Überwachungseinheit 37, insbesondere an das Rechenmodul, mittels einer internen Datenübertragungseinheit der Magnetresonanzvorrichtung 11 erfolgen. Zudem kann das Bereitstellen der Positionsdaten auch ein Bereitstellen an die externe Benutzerschnittstelle 38 der Überwachungseinheit 37 umfassen. Dabei kann das Bereitstellen auch ein Herstellen und/oder ein Aufbau einer Verbindung, insbesondere einer Datenverbindung, zu der externen Benutzerschnittstelle 38 und eine Datenübertragung an die externe Benutzerschnittstelle 38 umfassen. Das Bereitstellen der Positionsdaten an der externen Benutzerschnittstelle 38 umfasst bevorzugt auch ein Darstellen und/oder Anzeigen der Positionsdaten an der Ausgabeeinheit der externen Benutzerschnittstelle 38. Insbesondere kann derart ein externer Benutzer ein Video zur Positionierung des Patiententischs 16 und/oder des auf dem Patiententisch 16 positionierten Objekts 28 ansehen.

In einem daran anschließenden dritten Verfahrensschritt S3 erfolgt ein Bestimmen zumindest eines Einstellparameters für eine Einstellung der Position des Patiententischs 16 und/oder des auf dem Patiententisch 16 positionierten Objekts 28 anhand der erfassten und bereitgestellten Positionsdaten. Umfasst die Überwachungseinheit 37 eine externe Benutzerschnittstelle 38, die aktuell mit der Magnetresonanzvorrichtung 11 über die Schnittstellenmodule 41, 43 verbunden ist, wird von einem externen Benutzer an der externen Benutzerschnittstelle 38, insbesondere mittels der Eingabeeinheit 40, zumindest ein Einstellparameter zur Einstellung der Position des Patiententischs 16 und/oder des auf dem Patiententisch 16 positionierten Objekts 28 anhand der Positionsdaten ausgewählt. Besteht dagegen keine Verbindung zur externen Benutzerschnittstelle 38, kann von dem Rechenmodul der Überwachungseinheit 37 anhand der bereitgestellten Positionsdaten automatisch und/oder selbsttätig der zumindest einen Einstellparameter ermittelt und/oder bestimmt werden.

In einem weiteren Verfahrensschritt S4 erfolgt ein Bereitstellen des zumindest einen Einstellparameters an die Einstelleinheit 32 der Magnetresonanzvorrichtung 11. Dabei kann das Bereitstellen des zumindest einen Einstellparameters eine Übertragung des zumindest einen Einstellparameters von der Überwachungseinheit 37, insbesondere dem Rechenmodul der Überwachungseinheit 37, an die Einstelleinheit 32 mittels einer internen Datenübertragungseinheit der Magnetresonanzvorrichtung 11 erfolgen. Zudem kann das Bereitstellen des zumindest einen Einstellparameters an die Einstelleinheit 32 auch eine Übertragung des zumindest einen Einstellparameters von der externen Benutzerschnittstelle 38 der Überwachungseinheit 37 an die Einstelleinheit 32 mittels der durch die beiden Schnittstellenmodule 41, 43 aufgebauten Datenverbindung erfolgen.

In einem fünften Verfahrensschritt S5 erfolgt dann ein Einstellen der Position des Patiententischs 16 und/oder des auf dem Patiententisch 16 positionierten Objekts 28 mittels der Einstelleinheit 32 anhand des bereitgestellten Einstellparameters.

Die Verfahrensschritte S1 bis S5 werden dabei so oft wiederholt, bis der relevante und/oder mittels des Markierungselements 34 markierte Bereich des Patiententischs 16 und/oder des auf dem Patiententisch 16 positionierten Objekts 28 in der korrekt Referenzposition angeordnet und/oder positioniert ist.

Zudem können nach einer Positionierung des Patiententischs 16 und/oder des auf dem Patiententisch 16 positionierten Objekts 28 weiterhin Positionsdaten des Patiententischs 16 und/oder des auf dem Patiententisch 16 positionierten Objekts 28 in einem weiteren Verfahrensschritt S6 von der Positionserfassungseinheit 27, insbesondere der Kamera, erfasst werden, bis der Patiententisch 16 und/oder das auf dem Patiententisch 16 positionierte Objekt 28 in dem Isozentrum der Magnetresonanzvorrichtung 11 angeordnet ist. Diese in dem weiteren Verfahrensschritt S6 erfassten Positionsdaten werden ebenfalls der Überwachungseinheit 37 bereitgestellt, so dass der gesamte Einfahrvorgang und/oder der gesamte Positionierungsvorgang des Patiententischs 16 in den Patientenaufnahmebereich 13 insbesondere in das Isozentrum der Magnetresonanzvorrichtung 11, überwacht und kontrolliert werden kann. Derart können beispielweise mittels der Überwachungseinheit 37 von dem Patiententisch 16 herabhängende Gegenstände, wie beispielweise Kabel usw., erkannt werden. In einem derartigen Fall kann eine Bewegung des Patiententischs 16 sofort gestoppt werden, beispielweise durch ein manuelles Stoppen mittels der Eingabeeinheit 40 der externen Benutzerschnittstelle 38 oder ein automatisches Stoppen mittels des Rechenmoduls der Überwachungseinheit 37.

Nach einer Positionierung des Patiententischs 16 und/oder des auf dem Patiententisch 16 positionierten Objekts 28 innerhalb des Patientenaufnahmebereichs 13, insbesondere innerhalb des Isozentrums der Magnetresonanzvorrichtung 11, wird die Positionserfassungseinheit 27, insbesondere die Kamera, in einem weiteren, siebten Verfahrensschritt S7 deaktiviert. Zudem kann in dem Verfahrensschritt S7 die Positionserfassungseinheit 27, insbesondere die Kamera, bereits deaktiviert werden, sobald der Patiententisch 16 und/oder das auf dem Patiententisch 16 positionierte Objekt 28 innerhalb der Referenzposition angeordnet und/oder positioniert ist.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. System umfassend eine Magnetresonanzvorrichtung und eine Überwachungseinheit,
wobei die Magnetresonanzvorrichtung umfasst:
- eine Scannereinheit, die zu einer Erfassung von medizinischen Magnetresonanzdaten ausgebildet ist,
- einen von der Scannereinheit zumindest teilweise umgebenen Patientenaufnahmebereich,
- eine Patientenlagerungsvorrichtung mit einem bewegbaren Patiententisch, wobei der Patiententisch in den Patientenaufnahmebereich einfahrbar ausgebildet ist,
- eine Positionserfassungseinheit, die zu einer Erfassung von Positionsdaten einer Position des Patiententischs und/oder eines auf dem Patiententisch positionierten Objekts ausgebildet ist, wobei die Positionierungseinheit eine Kamera umfasst, und
- eine Einstelleinheit, die zu einer Einstellung der Position des Patiententischs und/oder des auf dem Patiententisch positionierten Objekts anhand eines bereitgestellten Einstellparameters ausgebildet ist,
wobei die Überwachungseinheit zu einer Bereitstellung eines Einstellparameters zu einer Einstellung einer Position des Patiententischs und/oder des auf dem Patiententischs positionierten Objekts anhand der erfassten Positionsdaten ausgebildet ist.

2. System nach Anspruch 1,
**dadurch gekennzeichnet, dass** die Magnetresonanzvorrichtung ein Schnittstellenmodul umfasst, das eine Schnittstelle aufweist und zu einem Aufbau einer Verbindung mit einem Schnittstellenmodul der Überwachungseinheit ausgebildet ist, und die Überwachungseinheit eine externe Benutzerschnittstelle umfasst, mit:
- einem Schnittstellenmodul, das eine Schnittstelle aufweist und zu einem Aufbau einer Verbindung mit einem Schnittstellenmodul der Magnetresonanzvorrichtung ausgebildet ist,
- einer Ausgabeeinheit, die zu einer Ausgabe der Positionsdaten ausgebildet ist, und
- einer Eingabeeinheit, die zu einer Eingabe eines Einstellparameters zu einer Einstellung einer Position des Patiententischs und/oder des auf dem Patiententisch positionierten Objekts ausgebildet ist,

3. System nach einem der vorhergehenden Ansprüche,
**dadurch gekennzeichnet, dass** die Scannereinheit eine Frontseite und eine Heckseite mit jeweils einer Einführöffnung für den Patientenaufnahmebereich umfasst und die Positionserfassungseinheit an der Frontseite und/oder an der Heckseite der Scannereinheit oberhalb der Einführöffnung angeordnet ist.

4. System nach einem der vorhergehenden Ansprüche,
**dadurch gekennzeichnet, dass** die Positionserfassungseinheit eine Datenschnittstelle aufweist für eine Verbindung mit der Einstelleinheit.

5. System nach einem der vorhergehenden Ansprüche,
**dadurch gekennzeichnet, dass** die Positionserfassungseinheit ein Markierungselement aufweist, das zu einer Markierung eines relevanten Bereichs des Patiententisch und/oder des auf dem Patiententischs positionierten Objekts ausgebildet ist.

6. System nach einem der vorhergehenden Ansprüche,
**dadurch gekennzeichnet, dass** die Positionserfassungseinheit, insbesondere die Kamera, eine Kalibriervorrichtung aufweist.

7. Verfahren zu einem Einstellen einer Position eines Patiententischs und/oder eines auf dem Patiententisch positionierten Objekts in einem System, das nach einem der Ansprüche 1 bis 6 ausgebildet ist, umfassend die folgenden Verfahrensschritte:
- S1: Erfassen von Positionsdaten des Patiententischs und/oder des auf dem Patiententisch positionierten Objekts mittels einer Positionserfassungseinheit einer Magnetresonanzvorrichtung, wobei die Positionsdaten zumindest teilweise Videodaten umfassen,
- S2: Bereitstellen der erfassten Positionsdaten an eine Überwachungseinheit,
- S3: Bestimmen zumindest eines Einstellparameters für eine Einstellung einer Position des Patiententischs und/oder des auf dem Patiententisch positionierten Objekts mittels der Überwachungseinheit anhand der erfassten Positionsdaten,
- S4: Bereitstellen des zumindest einen Einstellparameters an eine Einstelleinheit, und
- S5: Einstellen der Position des Patiententischs und/oder des auf dem Patiententisch positionierten Objekts mittels der Einstelleinheit anhand des zumindest einen bereitgestellten Einstellparameters.

8. Verfahren nach Anspruch 7,
**dadurch gekennzeichnet, dass** die Verfahrensschritte S1 bis S5 so oft wiederholt werden, bis der Patiententisch und/oder das auf dem Patiententisch positionierte Objekt in einer Referenzposition angeordnet ist.

9. Verfahren nach einem der Ansprüche 7 bis 8,
**dadurch gekennzeichnet, dass** während eines gesamten Einfahrvorgangs des Patiententischs in den Patientenaufnahmebereich von der Positionserfassungseinheit Positionsdaten erfasst und der Überwachungseinheit bereitgestellt werden.

10. Verfahren nach einem der Ansprüche 7 bis 9,
**dadurch gekennzeichnet, dass** die Positionserfassungseinheit während einer Magnetresonanzmessung deaktiviert wird.

11. Verfahren nach einem der Ansprüche 7 bis 10,
**dadurch gekennzeichnet, dass** die Überwachungseinheit ein Rechenmodul aufweist, das anhand der bereitgestellten Positionsdaten automatisch und/oder selbsttätig den zumindest einen Einstellparameter ermittelt.

12. Verfahren nach einem der Ansprüche 7 bis 11,
**dadurch gekennzeichnet, dass** die Überwachungseinheit eine externe Benutzerschnittstelle mit einem Schnittstellenmodul umfasst, wobei das Schnittstellenmodul zu einem Übertragen der Positionsdaten und/oder des zumindest einen Einstellparameters eine Datenverbindung mit der Magnetresonanzvorrichtung eingeht.

13. Computerprogrammprodukt, welches ein Programm umfasst und direkt in einen Speicher einer programmierbaren Steuereinheit ladbar ist, mit Programmmitteln, um ein Verfahren zu einem Einstellen einer Position eines Patiententischs und/oder eines auf dem Patiententisch positionierten Objekts nach einem der Ansprüche 7 bis 12 zu steuern, wenn das Programm in der Steuereinheit ausgeführt wird.
